# EUROPEAN PATENT SPECIFICATION

(11) **EP 1 383 461 B1**
(45) Date of publication and mention of the grant of the patent: **04.05.2011**
(21) Application number: 01995521.0
(22) Date of filing: 15.12.2001
(51) Int. Cl.: A61K 6/00, A61C 5/02, A61C 1/00, A61L 2/03

(54) **A COOLANT AND IRRIGANT FOR USE IN DENTAL SURGERY**
KÜHLMITTEL UND IRRIGATIONSMITTEL ZUR VERWENDUNG IN DER DENTALCHIRURGIE
FLUIDE DE REFROIDISSEMENT ET D'IRRIGATION UTILISE DANS LA CHIRURGIE DENTAIRE

(30) Priority: 15.12.2000 ZA 200007540; 15.12.2000 ZA 200007541; 15.12.2000 ZA 200007542; 15.12.2000 ZA 200007543
(43) Date of publication of application: 28.01.2004
(73) Proprietor: Radical Waters IP (PTY) Ltd, Midrand 1685 (ZA)
(72) Inventor: MARAIS, Jacobus Theodorus, Pretoria (ZA)
(74) Representative: Craven, Ian
(86) International application number: PCT/ZA2001/000206
(87) International publication number: WO 2003/088930

(56) References cited:
- WO-A-01/56616
- WO-A-02/04032
- WO-A-97/19707
- WO-A-99/34652
- US-A- 6 007 686

## Description

This invention relates to an irrigant for use in periodontal and surgical procedures involving alveolar bone, in surgical removal of teeth, during first stage implant surgery and in the treatment of post-extraction alveolar osteitis, as well as to a method for such surgical procedures, using such coolant and irrigant.

Simple extraction is the preferred, easy method of removal of teeth. In certain cases, such as impacted teeth, fractured teeth, or ankylosed teeth this is not possible and it is then necessary to surgically remove these teeth. Surgery includes the steps of the incision of the periodontal tissues and periosteum; the reflection of a full thickness flap (of periodontium and periosteum); the removal of the overlying bone by means of motor or turbine driven rotary instruments; and the elevating of the tooth or root. The process is completed with the appositioning and suturing of the soft tissues.

This procedure is typically followed by intense pain and swelling. The pain and swelling quite often lead to many days of bed rest and loss of productivity. One of the most common complications following the extraction of teeth is the infection of the tooth socket, commonly referred to as "dry socket". This condition is also known as alveolar osteïtis, alveolitis sicca dolorosa, alveolalgia, postoperative osteïtis or localised acute alveolar osteomyelitis.

In this condition, the blood clot covering the alveoli disintegrates or disappears, with the subsequent release of a foul odour and severe pain, but no suppuration. The exact cause of "dry socket" is not known. The nature of the pain is quite severe and healing is relatively slow. There is no standard regimen of treatment for the condition. Many modalities and medicines have been suggested but there had been, until now, no satisfactory method or agent for treatment of post-extraction alveolar osteïtis.

In certain cases of advanced adult periodontitis surgical treatment is indicated. Such surgical treatment usually involves incision of the periodontal and mucosal tissues, reflection of a full thickness flap, resection and contouring of the alveolar bone, appositioning of the flap and suturing of the wound. Resection and contouring of the bone, when done by means of motor or turbine driven rotary instruments such as dental drills must always be accompanied by copious cooling and irrigation with sterile water or saline. Failure to do so will result in necrosis of bone, delaying healing. Even with proper cooling and irrigation, severe post-operative pain and slow healing is quite common after periodontal surgery.

The use of osseo-integrated titanium implants in the jaws to support dental prostheses was pioneered by Branemark in Sweden. During the first stage of treatment, the bony mandible or maxilla is surgically exposed by incising the periodontium overlying the edentulous area and by reflecting a well-designed lap. The alveolar bone is then fenestrated in the exact position where the implant should be positioned by means of a sterile pilot drill driven by an electric motor, under sterile conditions. The pilot cavity is then reamed wider by means of another special bone-cutting drill. The surface area of this cavity is then counter sunk by means of a special countersinking drill. During the course of these drilling operations it is imperative that the drills and bone be cooled by a sterile, biocompatible solution. It is known that even the slightest increase in temperature or contamination by micro-organisms can lead to the eventual failure of the implant.

After the completion of the drilling process the implant is inserted into the cavity, the periodontal tissues appositioned and sutured. The implants are normally left undisturbed for several months and surgically exposed during second stage treatment and the dental prostheses fitted. Any failure of implant supported prostheses imply very serious, dire, clinical, practical, ethical, financial and legal consequences.

In normal dental practice, sterile water is used as coolant and irrigant. This is fed via an external line, a cumbersome device. Under no circumstance should tap water be used for cooling and irrigation and even more importantly, (prior to this invention) no coolant should be delivered by means of a water line integrated in the dental unit. It is known that most dental unit water lines are heavily contaminated by bacteria in the form of a biofilm. Tap water also contains micro-organisms.

WO-A-99/34652 discloses the use of an electrochemically activated aqueous solution from use in the treatment of root canals.

WO-A-01/56616 discloses dental equipment incorporating electrochemical apparatus.

### OBJET OF THE INVENTION

It is accordingly an object of this invention to provide a novel, relatively inexpensive and safe irrigant for use in dental surgery, including procedures such as the surgical removal of teeth, the treatment of alveolar osteitis, periodontal surgical procedures involving alveolar bone, first stage implant treatment surgery.

The term "dental surgical procedures" for purposes of this specification shall be interpreted as to include the surgical removal of teeth, periodontal surgical procedure involving alveolar bone, first stage implant treatment surgery and the treatment of alveolar osteitis, and cognate terms shall have corresponding meanings.

According to the invention there is provided the use of an an irrigant as described in claim 1.

The aqueous solution may consist of a mixture of an electrolytically activated, anion-containing solution and/or an electrolytically activated, cation-containing solution. The aqueous solution may consist of a mixture of the electrolytically activated, anion-containing solution and the electrolytically activated, cation-containing solution in the proportions and the state as produced by a suitable electrolytic device, capable of producing separate electrolytically activated, anion-containing and electrolytically activated, cation-containing solutions. The aqueous solution preferably consists of a mixture in a ratio of about 4 to 5 volumes of the electrolytically activated, anion-containing solution to about 1 volume of the electrolytically activated, cation-containing solution, when prepared from a n aqueous solution of a chloride or halide salt, and it would preferably consist of a mixture in a ratio of about 2 to 3 volumes of the electrolytically activated, anion-containing solution to about 1 volume of the electrolytically activated, cation-containing solution, when prepared from an aqueous solution of a bicarbonate or carbonate salt .

The anion-containing solution and/or the cation-containing solution may be prepared by means of electrolysis of an aqueous solution of a salt. The salt may be sodium chloride or sodium bicarbonate, or sodium carbonate. In particular, it may be non-iodised sodium chloride or potassium chloride or the bicarbonate or carbonate salt of sodium or potassium.

The anion-containing solution, produced in the anodic chamber of the electrolytic device, is referred to hereinafter as the "anolyte solution" or the "anolyte" and the cation-containing solution, produced in the cathodic chamber of the electrolytic device, is referred to hereinafter as the "catholyte solution" or the "catholyte".

The electrolytic device may include an electrolytic cell with predetermined design, geometrical and fluid flow relationships, ensuring optimum fluid flow and recirculation patterns. The cell may have a relatively small, annular, cross-sectional total open area for fluid flow, preferably of about 90 mm², thus causing turbulent fluid flow there through, so as to ensure maximum exposure of the solutions to the electric field.

The cell may be a through flow, electrolytic cell with two co-axial cylindrical electrodes, with a tubular ceramic diaphragm located co-axially between the two electrodes, so as to separate an annular inter-electrode space into a co-axial, annular catholytic and an annular anolytic chamber arrangement.

The electrolytic cell is preferably suitable to operate under predetermined operational parameters, including a relatively low current of about 1 to 15 A and preferably of about 5 to 7 A, and a relatively high voltage of about 1 to 48 V, preferably of about 6 to 18 V, and more preferably of 12 V, thus providing a relatively high voltage gradient or electric field strength at the interface between the electrode surface and electrolyte, estimated to be about 10⁶ V/cm.

The microcidal solution for use in the dental unit may be produced from an aqueous NaCl or NaHCO₃ or Na₂CO₃ solution, the concentration of which may vary between 0,01 % to 1 % and more specifically between 0.05 % and 0.5% and preferably between 0.1 % and 0.4%, electrolysed to produce cationic and anionic radicals.

The anolyte solution may have a redox potential of about + 200 to + 1100 mV and more specifically about + 600 to + 850 mV and preferably equal or more than + 713mV and a TDS of about 2-4 g/l. The anolyte solution may have a pH of about 6.75 to 8.5, preferably about 7.0 to 7.6, and a conductivity of about 0.1 to 10 mS/cm and more specifically of about 0.15 to 4.08 mS/cm, being produced at a current of about 5 Amperes, a voltage of about 12V and a flow rate of about 200 to 500 ml/min and more specifically about 300 to 350 ml/min. The anolyte solution may include species such as ClO; ClO⁻; HClO; OH⁻; HO₂⁻ ; H₂O₂; O₃; S₂O₈²⁻; and Cl₂O₆²⁻.

The above radicals in the anolyte solution have been found to have a suitable synergistic anti-microbial effect against viral organisms, spore and cyst-forming bacteria, fungi and yeast, which compares favourably with sodium hypochlorite and have been found to be particularly effective against Prevotella intermedia, Porphyromonas gingivalis, Streptococcus mutans and Enterococcus faecalis.

The catholyte solution may have a pH of about 9.0 - 12.0 and a redox potential of about -864 mV and a conductivity of about 5.92 to 6.03 mS/cm. The catholyte solution may include species such as NaOH; KOH; Ca(OH)₂; Mg(OH)₂; HO⁻; H₃O₂; HO₂; H₂O⁻₂; O₂⁻; OH⁻; and O₂²⁻.

It is believed that in addition to the normal mechanisms of action involved in elimination of micro-organisms, the oxidising free radicals and other constituents, such as micro-bubbles, present in the anolyte solution act synergistically at a bacterial cellular level, also aiding in the elimination of the micro-organisms in an electrostatic manner.

The efficacy of the mixed anolyte and catholyte solution as an irrigating medium for use in specific procedures, such as in the treatment of cavities and/or root canals, may depend upon the concentration of the mixed anolyte and catholyte solution in the receiving water, as measured by the pH, oxidation-reduction potential (ORP), conductivity and TDS of the mixed anolyte and catholyte solution, the exposure time, such as the contact time between the cavity and/or root canal, and the mixed anolyte and catholyte solution and the temperature during application.

### DETAILED DESCRIPTION OF THE INVENTION

Preferred embodiments of the invention will now be described by means of four non-limiting examples.

### EXAMPLE 1 :

Twenty patients requiring the surgical removal of teeth volunteered for this study. They were randomly assigned to one of two groups, Group A and Group B, each including ten patients. The same procedures were followed for both groups except for the coolant medium. In both groups local anaesthesia was administered, full thickness flaps designed, incisions made, flaps reflected, bone removed with rotary burs driven by electrical motors, teeth elevated, flaps repositioned and sutured.

### Group A (Saline)

In Group A the burs of rotary instruments were cooled by sterile saline delivered with a sterile chip syringe.

### Group B (Electrolytically Activated Solution "STEDS")

In Group B the burs were cooled with electrolytically activated water ("STEDS") delivered as an integrated spray through the normal integrated dental unit water lines, confirmed to be free of biofilm contamination.

STEDS was produced from a specially manufactured electrolytical reactor, comprising a through flow, electrolytical cell having two co-axial cylindrical electrodes with a co-axial diaphragm between them so as to separate an annular inter-electrode space into cathodic and anodic chambers. The STEDS produced included two separate solutions, namely catholyte and anolyte solutions. The anolyte solution had a pH of about 7.4 and a redox potential of about +1170 mV. The catholyte solution had a pH of about 9,5 and a redox potential of about -980mV.

### RESULTS

All patients were recalled and re-examined at 24 hours, 48 hours, 72 hours and one week post operatively. All complaints, signs and symptoms were categorised and classified according to a specially developed scale.

### Group A

In Group A all patients experienced severe pain, swelling and discomfort at 24 hours, 48 hours, as well as at 72 hours. At one week, 4 of the patients were still experiencing severe symptoms with the other 6 patients experiencing none.

### Group B

In Group B 4 patients experienced mild pain and discomfort at 24 hours. The other 6 experienced no pain. At 48 hours only one patient experienced mild discomfort. At 72 hours all patients were classified fully recovered. It was concluded that electrolytically activated water is superior to sterile saline as coolant and irrigant medium for the surgical removal of teeth.

### EXAMPLE 2 :

Twenty patients who reported for treatment of alveolar osteïtis volunteered for this study. Informed consent was obtained and the patients randomly assigned to one of two groups, Group A and B.

### Group A (Saline)

In Group A, treatment consisted of the surgical curettage and lavage with sterile saline, under local anaesthesia.

### Group B (Electrolytically Activated Solution "STEDS")

In Group B, treatment consisted of surgical curettage and lavage, using electrolytically activated solution ("STEDS"), also under local anaesthesia. The anolyte and catholyte solutions were used alternately. STEDS was produced from a specially manufactured electrolytical reactor, comprising a through flow, electrolytical cell having two co-axial cylindrical electrodes with a co-axial diaphragm between them so as to separate an annular inter-electrode space into cathodic and anodic chambers. The STEDS produced included two separate solutions, namely catholyte and anolyte solutions. The anolyte solution had a pH of about 7.4 and a redox potential of about +1170 mV. The catholyte solution had a pH of about 9,5 and a redox potential of about -980mV.

### RESULTS

All patients were re-examined at 24 hours, 48 hours, 72 hours and one week post-operatively and signs, symptoms and experience of pain and discomfort noted according to a special scale.

### Group A

In Group A, all patients were still experiencing severe pain at 24 as well as 48 hours. At 72 hours, five of the ten patients in Group A was still experiencing severe pain and discomfort. At one week post operatively three patients were still experiencing moderate to severe pain.

### Group B

In Group B, at 24 hours seven patients were totally free of pain and discomfort and were diagnosed as completely healed from alveolar osteïtis. The remaining three patients were still experiencing mild to moderate pain but at 48 hours they were also free from pain and were classified as healthy. It was concluded that treatment with electrolytically activated water is an effective, novel, safe and inexpensive method for the treatment of alveolar osteïtis.

### EXAMPLE 3:

Sixteen patients requiring periodontal surgery comprising of flap dissection, crown lengthening and or alveolar bone re-contouring procedures volunteered for this study. They were randomly assigned to one of two groups, Group A and Group B. One operator performed all sixteen operations. In Group A the rotary instruments were cooled with sterile saline and in Group B with electro-emically activated aqueous solutions. The patients were clinically re-assessed at 24 hours, 1 week later and six weeks post-operatively and at these times the patients were also asked to complete a specially developed questionnaire using a visual analog scale, to quantify their individual subjective experiences of pain. The results were statistically computed and analyzed using students t-test. In Group A all patients suffered severe post-operative pain at 24 hours and 6 of the 8 patients also suffered the same condition at one week post-operatively. Two patients were still suffering moderate pain at 6 weeks post-operatively. In Group B all patients were suffering only mild post-operative pain at 24 hours post-operatively. At one week post-operatively all patients were found to be totally pain free. It was concluded that electrolytically activated water when used as irrigant in periodontal surgical procedures involving alveolar bone resulted in significantly lower levels of post-operatively pain and swelling than when sterile saline was used.

### EXAMPLE 4 :

Nine healthy patients needing implant supported prostheses volunteered for this study and were randomly divided into three Groups namely, Groups A, B and C, comprising three patients each. Informed consent was obtained from the patient and treatment carried out.

### Group A (Saline)

In Group A first stage surgery was carried out in the conventional way using sterile saline delivered via an external line fitted to an electric motor driven instrument.

### Group B (Electrolytically Activated Solution "STEDS")

In Group B first stage surgery was carried out using electrolytically activated water ("STEDS") delivered via an external line fitted to the electric motor driven instrument.

### Group C

In Group C first stage surgery was carried out using electrolytically activated water ("STEDS") delivered directly into and through the electric motor which formed an integral part of a dental unit used in the surgery. This unit was confirmed to be free of biofilm contamination. In Group C, the electrolytically activated water had, in other words, been delivered via the normal dental water lines. Both in Group B and Group C, the STEDS was produced from a specially manufactured electrolytical reactor, comprising a through flow, electrolytical cell having two co-axial cylindrical electrodes with a co-axial diaphragm between them so as to separate an annular inter-electrode space into cathodic and anodic chambers. The STEDS produced included two separate solutions, namely catholyte and anolyte solutions. The anolyte solution had a pH of about 7.4 and a redox potential of about +1170 mV. The catholyte solution had a pH of about 9,5 and a redox potential of about-980mV. In both Groups B and C these two solutions were used alternately.

### RESULTS

First stage surgery was completed as usual and the patients followed up at 24 hours, 1 week, 1 month, 3 months and 6 months.

### Group A

At 24 hours all three patients in Group A experienced severe pain.

### Groups B and C

Patients in Groups B and C experienced no pain. At six months post-operatively second stage surgery was performed and prostheses made and fitted. Three months after that, all implants were found to be stable and functioning and the same was found one year after treatment had been completed. It was concluded that the use of electrolytically activated water was at least as good, possibly better than conventional coolant and irrigants. What was significant was that the use of electrolytically activated water in an integrated dental unit water line was equally effective as when used in an external separate special sterile water line and that it was equally effective as sterile saline delivered in such an external separate water line. This represents a significant saving in equipment and costs.

## Claims

1. Use of an irrigant obtainable by means of electrolysis of a dilute saline solution of 0.01 to 1% thus minimising the formation of trihalomethanes and therefore the carcinogenic potential of the solution, said irrigant comprising: an electrolytically activated aqueous solution selected from a group consisting of: a mixture of an anion-containing solution and a cation-containing solution; an anion-containing solution obtainable from a cation-containing solution or a mixture of an anion-containing solution and a cation-containing solution; and a cation-containing solution obtainable from an anion-containing solution, a cation-containing solution or a mixture of an anion-containing solution and a cation-containing solution; and wherein the anion-containing solution has a pH of 6.75 to 10 and the cation-containing solution has a pH of 9.0 - 12.0, such that the electrolytically activated aqueous solution has a pH of between 6.75 and 10 with microcidal as well as anti-oxidising, dispersing and surfactant properties for rendering the irrigant bio-compatible and non-corrosive in the manufacture of a medicament for use in dental surgical procedures.

2. Use as claimed in claim 1 wherein the irrigant consists of a mixture of electrolytically activated, anion-containing solution and electrolytically activated cation-containing solution in the proportions and the state obtainable by generation from an electrolytic device capable of producing separate electrolytically activated, anion containing and electrolytically activated cation containing solutions, the irrigant consisting of a mixture in a ratio of 4 to 5 volumes of the electrolytically activated, anion-containing solution to 1 volume of the electrolytically activated cation-containing solution obtainable from an aqueous solution of a chloride or halide salt.

3. Use as claimed in claim 1 wherein the irrigant consists of a mixture of electrolytically activated, anion-containing solution and electrolytically activated cation-containing solution in the proportions and the state obtainable by generation from an electrolytic device capable of producing separate electrolytically activated, anion-containing and electrolytically activated cation-containing solutions, the irrigant consisting of a mixture in a ratio of 2 to 3 volumes of the electrolytically activated, anion-containing solution to 1 volume of the electrolytically activated cation-containing solution obtainable from an aqueous solution of a bicarbonate or carbonate salt.

4. Use as claimed in claim 2 or 3 wherein the anion-containing solution has a redox potential of +200 to +1200mv, a TDS of 2-4g/l and a conductivity of 0.1 to 10mS/cm produced at a current of 2 to 15 amperes, a voltage of 2 to 48V and a flow rate of 200 to 500 ml/min.

5. Use as claimed in claim 4 wherein the anion-containing solution has any one or more of species selected from the group consisting of Cl₂, HClO, HCl, HO₂⁺, HO⁺, ClO⁻, H₂O₂, O₂, Cl⁺, HClO₂, ClO₂, O⁺, ClO, ClO⁻, HClO, OH⁻, H₂O₂, S₂O₈²⁻, ClO₃⁻ , Cl₂O₆²⁻, HO₂⁻ and O₃.

6. Use as claimed in claim 2 or 3 wherein the cation-containing solution has a redox potential of -864mV and a conductivity of 5.92 to 6.03 mS/cm.

7. Use as claimed in claim 6 wherein the cation-containing solution has any one or more of the species selected from the group consisting of NaOH, KOH, Ca(OH)₂, Mg(OH)₂, HO⁻, H₃O₂, HO₂⁻, O₂⁻, H₂O₂⁻ , OH⁻, O₂²⁻, NaOH, HO²⁺; OH⁺, H₂O₂, H⁺ and OH⁺.

8. Use as claimed in claim 1 wherein the dental surgical procedures include: irrigating the wound and dental surgical instruments with the medicament.

9. Use as claimed in claim 8 wherein the dental surgical procedures include the steps of:
surgical removal of teeth; and
irrigating the teeth and at least some of the associated alveoli and rotary instruments with the medicament.

10. Use as claimed in claim 8 wherein the dental surgical procedures include the steps of:
treating post-extraction alveolar osteitis; and
applying the medicament to affected alveoli.

11. Use as claimed in claim 8 wherein the dental surgical procedures include the steps of:
periodontal surgical procedure involving alveolar bone; and
irrigating at least some of the rotary instruments and the alveolar bone with the medicament.

12. Use as claimed in claim 8 wherein the dental surgical procedures include the steps of:
first stage implant treatment surgery; and
irrigating at least some of the dental surgical instruments, the alveolar bone, the basal bone and the implant components with the medicament.

## Patentansprüche

1. Verwendung eines Irrigationsmittels, das durch die Elektrolyse einer verdünnten Salzlösung von 0,01 bis 1 % erhältlich ist, wodurch die Bildung von Trihalogenmethanen auf ein Minimum eingeschränkt und dementsprechend das karzinogene Potential der Lösung minimiert wird, wobei das Irrigationsmittel Folgendes enthält: eine elektrolytisch aktivierte wässrige Lösung, die ausgewählt ist aus der Gruppe, bestehend aus :
einem Gemisch aus einer Anionen enthaltenden Lösung und einer Kationen enthaltenden Lösung; einer Anionen enthaltenden Lösung, die aus einer Kationen enthaltenden Lösung oder einem Gemisch von einer Anionen enthaltenden Lösung und einer Kationen enthaltenden Lösung erhältlich ist; und einer Kationen enthaltenden Lösung, die aus einer Anionen enthaltenden Lösung,
einer Kationen enthaltenden Lösung oder einem Gemisch aus einer Anionen enthaltenden Lösung und einer Kationen enthaltenden Lösung erhältlich ist; und wobei die Anionen enthaltende Lösung einen pH-Wert zwischen pH 6,75 und 10 und die Kationen enthaltende Lösung einen pH-Wert zwischen pH 9,0 und 12,0 aufweist,
sodass die elektrolytisch aktivierte wässrige Lösung einen pH-Wert zwischen pH 6,75 und 10 aufweist und mit mikrobiziden sowie antioxidativen, dispergierenden und
oberflächenaktiven Eigenschaften ausgestattet ist, um das Irrigationsmittel für die Herstellung von einem Medikament für die Verwendung bei zahnchirurgischen Verfahren biologisch verträglich und korrosionssicher zu machen.

2. Verwendung wie in Anspruch 1 beansprucht, wobei das Irrigationsmittel aus einem Gemisch von einer elektrolytisch aktivierten, Anionen enthaltenden Lösung und einer elektrolytisch aktivierten, Kationen enthaltenden Lösung in den Verhältnissen und der Beschaffenheit besteht, die durch die Erzeugung mit einer elektrolytischen Vorrichtung erhältlich ist, welche in der Lage ist, elektrolytisch aktivierte, Anionen enthaltende und elektrolytisch aktivierte, Kationen enthaltende Lösungen getrennt voneinander herzustellen, wobei das Irrigationsmittel aus einem Gemisch mit einem Verhältnis von 4 bis 5 Volumen der elektrolytisch aktivierten, Anionen enthaltenden Lösung zu 1 Volumen der elektrolytisch aktivierten, Kationen enthaltenden Lösung, die aus einer wässrigen Lösung eines Chlorid- oder Halogensalzes erhältlich ist, besteht.

3. Verwendung wie in Anspruch 1 beansprucht, wobei das Irrigationsmittel aus einem Gemisch von einer elektrolytisch aktivierten, Anionen enthaltenden Lösung und einer elektrolytisch aktivierten, Kationen enthaltenden Lösung in den Verhältnissen und der Beschaffenheit besteht, die durch die Erzeugung mit einer elektrolytischen Vorrichtung erhältlich ist, welche in der Lage ist, elektrolytisch aktivierte, Anionen enthaltende und elektrolytisch aktivierte, Kationen enthaltende Lösungen getrennt voneinander herzustellen, wobei das Irrigationsmittel aus einem Gemisch mit einem Verhältnis von 2 bis 3 Volumen der elektrolytisch aktivierten, Anionen enthaltenden Lösung zu 1 Volumen der elektrolytisch aktivierten, Kationen enthaltenden Lösung, die aus einer wässrigen Lösung eines Bicarbonat- oder Carbonatsalzes erhältlich ist, besteht.

4. Verwendung wie in Anspruch 2 oder 3 beansprucht, wobei die Anionen enthaltende Lösung ein Redoxpotential von +200 bis +1.200 mV, einen TDS-Wert von 2 - 4 g/l und eine Leitfähigkeit von 0,1 bis 10 mS/cm, erzeugt bei einer Stromstärke von 2 bis 15 Ampere, Einer Spannung von 2 bis 48 V und einer Flussrate von 200 bis 500 ml/min.

5. Verwendung wie in Anspruch 4 beansprucht, wobei die Anionen enthaltende Lösung eine oder mehrere der chemischen Substanzen enthält, die ausgewählt sind aus der Gruppe, die aus Cl₂, HClO, HCl, HO₂⁺, HO⁺, ClO⁻, H₂O₂, O₂, Cl⁺, HClO₂, ClO₂, O⁺, ClO, ClO⁻, HClO, OH⁻, H₂O₂, S₂O₈²⁻, ClO₃⁻, Cl₂O₆²⁻, HO₂⁻ und O₃ besteht.

6. Verwendung wie in Anspruch 2 oder 3 beansprucht, wobei die Kationen enthaltende Lösung ein Redoxpotential von -864 mV und eine Leitfähigkeit von 5,92 bis 6,03 mS/cm aufweist.

7. Verwendung wie in Anspruch 6 beansprucht, wobei die Kationen enthaltende Lösung eine oder mehrere der chemischen Substanzen enthält, die ausgewählt sind aus der Gruppe, die aus NaOH, KOH, Ca (OH)₂, Mg (OH)₂, HO⁻, H₃O₂, HO₂⁻, O₂⁻, H₂O₂⁻, OH⁻, O₂²⁻, NaOH, HO²⁺; OH⁺, H₂O₂, H⁺ und OH⁺ besteht.

8. Verwendung wie in Anspruch 1 beansprucht, wobei die zahnmedizinischen chirurgischen Verfahren einschließen: das Spülen der Wunde und der zahnmedizinischen chirurgischen Instrumente mit dem Medikament.

9. Verwendung wie in Anspruch 8 beansprucht, wobei die zahnmedizinischen chirurgischen Verfahren die Schritte einschließen von:
operatives Entfernen der Zähne; und
Spülen der Zähne und von mindestens einigen der zugehörigen Alveolen und der rotierend arbeitenden Instrumente mit dem Medikament.

10. Verwendung wie in Anspruch 8 beansprucht, wobei die zahnmedizinischen chirurgischen Verfahren die Schritte einschließen von:
Behandeln der postoperativen alveolären Ostitis; und
Anwenden des Medikaments auf die betroffenen Alveolen.

11. Verwendung wie in Anspruch 8 beansprucht, wobei die zahnmedizinischen chirurgischen Verfahren die Schritte einschließen von:
Parodontalchirurgie-Verfahren, das den Alveolarknochen einbezieht; und Spülen von mindestens einigen der rotierend arbeitenden Instrumente und des Alveolarknochens mit dem Medikament.

12. Verwendung wie in Anspruch 8 beansprucht, wobei die zahnmedizinischen chirurgischen Verfahren die Schritte einschließen von:
erster Abschnitt der chirurgischen Behandlung für das Implantat; und
Spülen von mindestens einigen der zahnmedizinischen chirurgischen Instrumente, des Alveolarknochens, des Basalknochens und der Implantat-Komponenten mit dem Medikament.

## Revendications

1. Utilisation d'un irrigant pouvant être obtenu par électrolyse d'une solution salée diluée de 0,01 à 1 % en minimisant ainsi la formation de trihalométhanes et donc le potentiel carcinogène de la solution, ledit irrigant comprenant : une solution aqueuse activée électrolytiquement choisie dans un groupe consistant en : un mélange d'une solution contenant des anions et d'une solution contenant des cations ; une solution contenant des anions pouvant être obtenue à partir d'une solution contenant des cations ou d'un mélange d'une solution contenant des anions et d'une solution contenant des cations ; et une solution contenant des cations pouvant être obtenue à partir d'une solution contenant des anions, d'une solution contenant des cations ou d'un mélange d'une solution contenant des anions et d'une solution contenant des cations ; et où la solution contenant des anions a un pH de 6,75 à 10 et la solution contenant des cations a un pH de 9,0-12,0, de sorte que la solution aqueuse activée électrolytiquement a un pH entre 6,75 et 10 avec des propriétés microbicides ainsi que des propriétés antioxydantes, dispersantes et tensioactives pour rendre l'irrigant biocompatible et non corrosif dans la fabrication d'un médicament destiné à être utilisé dans des interventions de chirurgie dentaire.

2. Utilisation selon la revendication 1 où l'irrigant consiste en un mélange de solution contenant des anions activée électrolytiquement et de solution contenant des cations activée électrolytiquement dans les proportions et l'état pouvant être obtenus par formation à partir d'un dispositif électrolytique capable de produire des solutions contenant des anions activées électrolytiquement et contenant des cations activées électrolytiquement séparées, l'irrigant consistant en un mélange dans un rapport de 4 à 5 volumes de la solution contenant des anions activée électrolytiquement à 1 volume de la solution contenant des cations activée électrolytiquement pouvant être obtenu à partir d'une solution aqueuse d'un sel chlorure ou halogénure.

3. Utilisation selon la revendication 1 où l'irrigant consiste en un mélange de solution contenant des anions activée électrolytiquement et de solution contenant des cations activée électrolytiquement dans les proportions et l'état pouvant être obtenus par formation à partir d'un dispositif électrolytique capable de produire des solutions contenant des anions activées électrolytiquement et contenant des cations activées électrolytiquement séparées, l'irrigant consistant en un mélange dans un rapport de 2 à 3 volumes de la solution contenant des anions activée électrolytiquement à 1 volume de la solution contenant des cations activée électrolytiquement pouvant être obtenu à partir d'une solution aqueuse d'un sel bicarbonate ou carbonate.

4. Utilisation selon la revendication 2 ou 3 où la solution contenant des anions a un potentiel redox de +200 à +1200 mv, un TDS de 2-4 g/l et une conductivité de 0,1 à 10 mS/cm produite à un courant de 2 à 15 ampères, une tension de 2 à 48 V et un débit de 200 à 500 ml/min.

5. Utilisation selon la revendication 4 où la solution contenant des anions a une ou plusieurs espèces quelconques choisies dans le groupe consistant en Cl₂, HClO, HCl, HO₂⁺, HO⁺, ClO⁻, H₂O₂, O₂, Cl⁺, HClO₂, ClO₂, O⁺, ClO, ClO⁻, HClO, OH⁻, H₂O₂, S₂O₈²⁻, ClO₃⁻, Cl₂O₆²⁻, HO₂⁻ et O₃.

6. Utilisation selon la revendication 2 ou 3 où la solution contenant des cations a un potentiel redox de -864 mV et une conductivité de 5,92 à 6,03 mS/cm.

7. Utilisation selon la revendication 6 où la solution contenant des cations a une ou plusieurs espèces quelconques choisies dans le groupe consistant en NaOH, KOH, Ca(OH)₂, Mg(OH)₂, HO⁻, H₃O₂, HO₂⁻, O₂⁻ , H₂O₂⁻, OH⁻, O₂²⁻, NaOH, HO²⁺; OH⁺, H₂O₂, H⁺ et OH⁺.

8. Utilisation selon la revendication 1 où les interventions de chirurgie dentaire incluent : l'irrigation de la plaie et des instruments de chirurgie dentaire avec le médicament.

9. Utilisation selon la revendication 8 où les interventions de chirurgie dentaire incluent les étapes de :
retrait chirurgical de dents ; et
irrigation des dents et d'au moins certains des alvéoles associés et des instruments rotatifs avec le médicament.

10. Utilisation selon la revendication 8 où les interventions de chirurgie dentaire incluent les étapes de :
traitement de l'alvéolite après l'extraction ; et
application à du médicament aux alvéoles affectés.

11. Utilisation selon la revendication 8 où les interventions de chirurgie dentaire incluent les étapes de :
intervention chirurgicale paradontale impliquant l'os alvéolaire ; et
irrigation d'au moins certains des instruments rotatifs et de l'os alvéolaire avec le médicament.

12. Utilisation selon la revendication 8 où les interventions de chirurgie dentaire incluent les étapes de :
chirurgie de traitement par implant de premier stade ; et
irrigation d'au moins certains des instruments de chirurgie dentaire, de l'os alvéolaire, de l'os basal et des composants de l'implant avec le médicament.
